# EUROPEAN PATENT APPLICATION

(11) **EP 3 404 566 A1**
(43) Date of publication of application: **21.11.2018**
(21) Application number: 17171190.6
(22) Date of filing: 15.05.2017
(51) Int. Cl.: G06F 19/00, A61B 5/00, A61B 5/103, G06K 9/62

(54) **SYSTEM AND METHOD FOR PROVIDING FOOT SHAPE AND MOTION CHARACTERISTICS**

(71) Applicant: RS Biomechanics BVBA, 3583 Beringen (BE)
(72) Inventor: WILSSENS, Jean-Pierre, 3583 Beringen (BE); HAGMAN, Friso, 3583 Beringen (BE)
(74) Representative: IPLodge bvba

(57) **Abstract**

The invention pertains to a method for training an expert system for assessing dynamic plantar pressure measurements, the method comprising: obtaining, for each subject of said group of subjects, an image comprising time-varying two-dimensional plantar pressure measurements of at least one foot; spatially registering said obtained images of respective subjects to a target image; temporally registering said obtained images of respective subjects to said target image; and deriving (150) a plurality of categories of said subjects by applying a clustering algorithm to sets of data points obtained at corresponding coordinates in said spatially and temporally registered images. The invention also pertains to a of using an expert system for assessing dynamic plantar pressure measurements of a subject, to a computer program product, and to an expert system.

## Description

The present invention pertains to systems and methods for characterizing foot shape and motion by means of plantar pressure measurements. In particular, the present application discloses an expert system, a method for training an expert system, and a method for using an expert system.

### Background

Plantar pressure measurements have been used *inter alia* to evaluate the impact of neurological diseases, diabetes, and surgical interventions. Such plantar pressure studies have relied on quantitative analysis techniques to ensure that their conclusions do not differ from clinician to clinician.

A typical plantar pressure image acquires measurements over a lattice with 4 sensors per square centimeter, while a dynamic measurement can typically acquire readings from those sensors at a rate of up to 500 Hz. As a result, a single dynamic plantar pressure image provides pressure readings over a three-dimensional space - incorporating pressure information along two spatial dimensions and a temporal dimension - and contains on the order of tens of thousands of activated sensors. To handle such a large volume of data, it has become customary to aggregate and subsample those measurements through the use of peak pressure images, center of pressure trajectories, or regions of interest based on anthropometric information of healthy subjects.

It is a disadvantage of the known methods that valuable information present in the full spatio-temporal pressure image is discarded. Accordingly, there is a need for systems that efficiently use the information present in the full image.

### Summary

According to an aspect of the present invention, there is provided a method for training an expert system for assessing dynamic plantar pressure measurements, the method comprising: obtaining, for each subject of a group of subjects, an image comprising time-varying two-dimensional plantar pressure measurements of at least one foot; spatially registering the obtained images of respective subjects to a target image; temporally registering the obtained images of respective subjects to the target image; and deriving a plurality of categories of the subjects by applying a clustering algorithm to sets of data points obtained at corresponding coordinates in the spatially and temporally registered images.

In the present text, the term "image" is used to denote a 4-dimensional data set, consisting of measured pressure values indexed by two spatial coordinates (along the dimensions of the pressure sensor grid) and a temporal coordinate (the progress of time from the beginning of a measurement to the end). An image thus comprises dynamic plantar pressure data, which reveals information about the way the pressure distribution varies over time through the different stages of a footstep.

The present invention is based *inter alia* on the insight of the inventors that the dynamic plantar pressure data contain very valuable diagnostic information, and that the dynamic plantar pressure data can be efficiently processed despite its huge size by applying adequate pre-processing techniques, in particular registering images to a target image to allow the application of clustering algorithms and other statistical processes on those images, which lead to categories and associated reference values that can efficiently be used to categorize subjects. In embodiments of the present invention, dynamic plantar pressure data acquired at a high resolution in both the spatial domain and the temporal domain can be accurately analyzed, despite the huge size of the data sets, by applying judiciously chosen processing methods that were hitherto not believed to be usable for this application.

In an embodiment, the training method according to the present invention further comprises: obtaining, for each subject of said group of subjects, a three-dimensional scan of said at least one foot during said obtaining of said image; spatially registering said obtained three-dimensional scans of respective subjects to a target model; temporally registering said obtained three-dimensional scans of respective subjects to said target model; and performing said deriving of said plurality of categories of said subjects by applying said clustering algorithm also to sets of features in said spatially and temporally registered three-dimensional scans.

It is an advantage of this embodiment that the determination of clusters and statistics can be performed more accurately by also taking into account three-dimensional information representing the deformation of the foot under examination during the measurement of the pressure image. This allows for a determination of correlations of certain pressure features with certain three-dimensional deformation features.

In an embodiment, the training method according to the present invention further comprises: obtaining, for each subject of said group of subjects, a time-dependent force plate measurement of said at least one foot during said obtaining of said image; temporally registering said obtained time-dependent force plate measurements of respective subjects to a target measurement; and performing said deriving of said plurality of categories of said subjects by applying said clustering algorithm also to sets of data points obtained at corresponding coordinates in temporally registered time-dependent force plate measurements.

It is an advantage of this embodiment that the determination of clusters and statistics can be performed more accurately by also taking into account force plate measurements representing the total force exerted by the foot under examination during the measurement of the pressure image, preferably along multiple axes (e.g., x-y-z force measurement). This allows for a determination of correlations of certain force features with certain three-dimensional deformation features.

In an embodiment of the training method according to the present invention, the temporally registering comprises using continuous dynamic time warping.

Continuous dynamic time warping attempts to match the pressure samples at one time point in the moving image to a weighted combination of pressure samples from neighboring time points in the target image. The matching is performed in a way that minimizes the sum-squared difference between the time samples while also ensuring that the plantar pressure samples in the moving image maintain their original temporal order. The inventors have found this technique is particularly useful for the purpose of the present invention. Further details can be found in F. Zhou and F.D. la Torre, "Generalized canonical time warping", IEEE Transactions on Pattern Analysis and Machine Intelligence 38 (2) (2016) 279-294.

In an embodiment of the training method according to the present invention, obtaining images of the subjects comprises: conducting a series of time-varying two-dimensional plantar pressure measurements for each subject of the group of subjects; spatially aligning the two-dimensional plantar pressure measurements by means of a rigid transformation; and obtaining the image by calculating an average image from the spatially aligned two-dimensional plantar pressure measurements.

By averaging out a series of measurements, certain measurement errors can be removed from the image (in particular, measurement errors that are independent and have a zero average). This technique requires at least two measurements, but the use of at least five measurements is preferred, and the use of at least ten measurements provides even more accurate results. In order to make sure that the relevant parts of the images are combined in the averaging operation, the two-dimensional plantar pressure measurements must be aligned in the space domain by means of a two-dimensional rigid transformation.

In a particular embodiment, spatially aligning the two-dimensional plantar pressure measurements comprises generating a peak-pressure image from each of the time-varying two-dimensional plantar pressure measurements, and deriving parameters of the rigid transformation from a spatial relationship between the peak-pressure images.

The inventors have found that a peak-pressure image is sufficiently representative for the image as a whole to be used as the basis for determining a rigid transformation that provides an adequate spatial transformation.

In an embodiment, the training method according to the present invention further comprises obtaining the target image by: selecting an intermediate target image; for each image, determining a registration transformation that registers the image to the intermediate target image; calculating a biased average image from the registered images; calculating an average transformation from the registration transformations; inverting the average transformation; and applying the average transformation to the biased average image to obtain the target image.

This embodiment is based on the insight of the inventors that it is best to perform clustering and statistical analysis on the basis of images that are registered to an anatomically neutral image. The embodiment is further based on the insight of the inventors that the measurements of the training population as a whole can be used to define what an anatomically neutral image is. The presently disclosed steps lead to the derivation of an anatomically neutral image by removing the bias introduced by an initially used "intermediate target image", which may be randomly selected.

In an embodiment of the training method according to the present invention, the group of subjects comprises humans. In another embodiment of the training method according to the present invention, the group of subjects comprises animals.

In an embodiment of the training method according to the present invention, the group of subjects comprises at least 1000 subjects.

The group of subjects used to train the expert system is preferably a group that displays a range of values of the measured characteristic which is representative of the population to which they belong. This may include subjects that might be considered anatomically normal as well as subjects that have been previously diagnosed with a deviation (e.g., people that already use prescribed orthopedic shoes or other forms of corrective support). It is an advantage of the method of the present invention that the "normal" values will be determined autonomously. The inventors have found that a group size of 1000 is typically sufficient to provide a sufficiently accurate expert system.

According to an aspect of the present invention, there is provided a method of using an expert system for assessing dynamic plantar pressure measurements of a subject, the expert system having been trained using time-varying two-dimensional plantar pressure measurements of a number of prior subjects, the method comprising: obtaining an image comprising time-varying two-dimensional plantar pressure measurements of at least one foot of the subject; spatially registering the obtained image to a target image; temporally registering the obtained images to the target image; and assigning the subject to one or more of a plurality of categories by comparing data points in the spatially and temporally registered image to reference values stored in the expert system.

Once the expert system has been set up by training, it may then be used to process actual patient images. The steps required to make the patient images comparable to the reference values are similar to the steps of the training method, and include spatial and temporal registration to a target image.

The expert system may be deployed as a web-based or cloud-based system (i.e., the system may be operated under the "software-as-a-service" model), whereby the time-varying two-dimensional plantar pressure measurements are uploaded to the expert system for processing via the internet or a similar network, and whereby the resulting classification is returned by the expert system to the client.

In an embodiment, the method of using an expert system according to the present invention further comprises: obtaining a three-dimensional scan of said at least one foot during said obtaining of said image; spatially registering said obtained three-dimensional scan to a target model; and temporally registering said obtained three-dimensional scan to said target model; and the assigning further comprises comparing data points in said spatially and temporally registered three-dimensional scan to reference values stored in said expert system.

The steps required to make the patient's three-dimensional scans comparable to the reference values are similar to the steps of the training method, and include spatial and temporal registration to a target model. The registered scans are also used for the categorization of the patient.

In an embodiment, the method of using an expert system according to the present invention further comprises: obtaining a time-dependent force plate measurement of said at least one foot during said obtaining of said image; and temporally registering said obtained time-dependent force plate measurement to a target measurement; and the assigning further comprising comparing data points obtained at corresponding coordinates in temporally registered time-dependent force plate measurements to reference values stored in said expert system.

The steps required to make the patient's force plate measurements comparable to the reference values are similar to the steps of the training method, and include temporal registration to a target measurement. The registered force plate measurements are also used for the categorization of the patient.

In an embodiment, the method according to the present invention further comprises: outputting one or more of a treatment option, a set of shoe parameters, a set of insole parameters, a bandage type, and a medication, associated with the one or more categories.

It is an advantage of embodiments of the present invention that the detectable anatomical categories can be associated with concrete actions that would benefit the patient, such as treatment options, corrective shoe and insole parameters, bandage types, medication, and the like.

In an embodiment of the method according to the present invention, obtaining the image comprises: conducting a series of time-varying two-dimensional plantar pressure measurements for each subject of a group of subjects; spatially aligning the two-dimensional plantar pressure measurements; and obtaining the image by calculating an average image from the aligned two-dimensional plantar pressure measurements.

By averaging out a series of measurements, certain measurement errors can be removed from the image (in particular, measurement errors that are independent and have a zero average). This technique requires at least two measurements, but the use of at least five measurements is preferred, and the use of at least ten measurements provides even more accurate results, or up to a point where a certain convergence criterion is reached (which may be signaled by the expert system itself). In order to make sure that the relevant parts of the images are combined in the averaging operation, the two-dimensional plantar pressure measurements must be aligned in the space domain by means of a two-dimensional rigid transformation.

In an embodiment, the method according to the present invention further comprises: updating the expert system by adding the obtained image to the time-varying two-dimensional plantar pressure measurements of the number of prior subjects.

It is an advantage of this embodiment that the expert system becomes more accurate over time, as more measurement data are added to the training set. This is particularly practical in web based or cloud based system, whereby the time-varying two-dimensional plantar pressure measurements obtained at a large number of measurement sites (e.g. clinics and hospitals) have to be uploaded to a centralized expert system for processing. The centralized expert system then has these measurements at its disposal for retraining.

In a particular embodiment, the updating is performed by adding batches of obtained images to the time-varying two-dimensional plantar pressure measurements of the number of prior subjects, when a criterion pertaining to the obtained images is met.

The criterion may take into account the number of obtained images, the number of obtained images that present a certain deviation from a selected standard value, or the like. It is an advantage of this embodiment that the updating process can be made more efficient, by initiating an update only when a sufficient amount of relevant new information is available for addition to the expert system.

In a particular embodiment, the updating is performed periodically by adding batches of obtained images to the time-varying two-dimensional plantar pressure measurements of the number of prior subjects.

It is an advantage of this embodiment that the downtime of the expert system due to reanalysis of the training data can be reduced. The batch updating may be performed for example at weekly, monthly, or quarterly intervals.

According to an aspect of the present invention, there is provided a computer program product comprising code means configured to make a processor carry out the steps of the method described above.

According to an aspect of the present invention, there is provided an expert system for assessing dynamic plantar pressure measurements of a subject, the expert system comprising: a learning module configured to learn by using time-varying two-dimensional plantar pressure measurements of a number of prior subjects; and an analysis module configured to analyze an image comprising time-varying two-dimensional plantar pressure measurements of a target subject; wherein the analysis module is configured to spatially and temporally register the obtained image to a target image and to assign the subject to one or more of a plurality of categories by comparing data points in the spatially and temporally registered image to reference values stored in the expert system.

The expert system may further be adapted to operate on three-dimensional scans and/or force plate measurements as disclosed hereinabove.

The technical effects and advantages of embodiments of the expert system and the computer program product according to the present invention correspond *mutatis mutandis* to those of the corresponding embodiments of the method according to the present invention.

### Description of the Figures

These and other features and advantages of embodiments of the present invention will now be described in more detail with reference to the accompanying drawings, in which:
- Figure 1 presents a flow chart of an embodiment of a method for training an expert system for assessing dynamic plantar pressure measurements according to the present invention; and
- Figure 2 presents a flow chart of an embodiment of a method for using an expert system for assessing dynamic plantar pressure measurements according to the present invention.

### Detailed Description of Embodiments

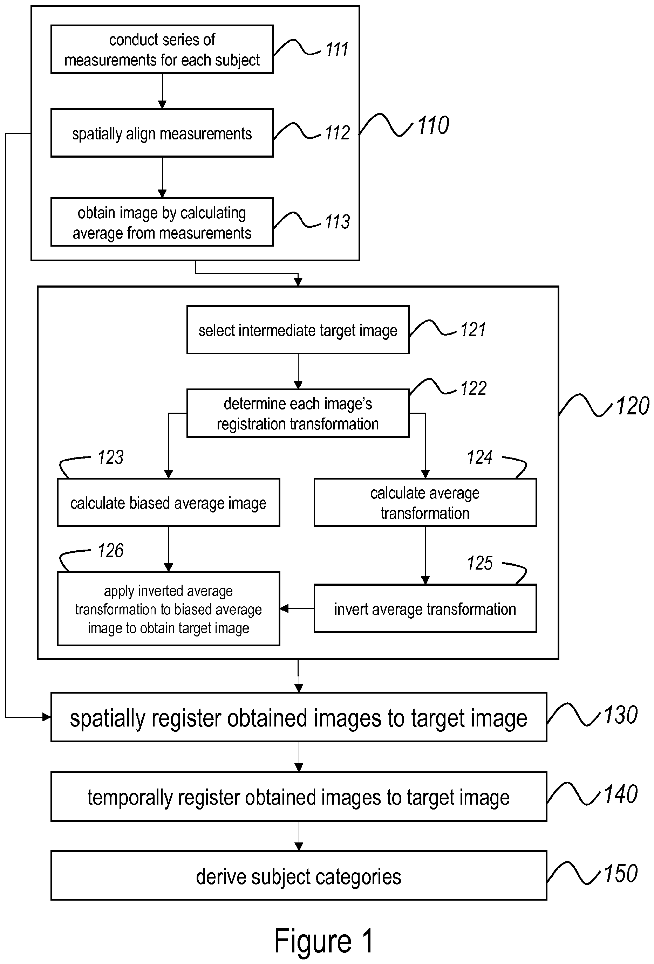

Figure 1 presents a flow chart of an embodiment of a method for training an expert system for assessing dynamic plantar pressure measurements according to the present invention.

The method comprises obtaining **110**, for each subject of the group of subjects (i.e., humans or animals), an image comprising time-varying two-dimensional plantar pressure measurements. The group of subjects preferably contains a large number of subjects, e.g. at least 100 subjects, preferably at least 500 subjects, and more preferably at least 1000 subjects. Obtaining the images may comprise conducting **111** a series of time-varying two-dimensional plantar pressure measurements for each subject of the group of subjects. For this purpose, the known "rs scan"® pressure plate may be used. Such a pressure plate has non-square sensor dimensions of 5.08 mm × 7.62 mm, resulting in plantar pressure images that are compressed in the vertical direction. To correct for the sensor dimensions, each plantar pressure image may be interpolated upward; good results have been obtained with a target grid of 1 mm × 1 mm, interpolated using cubic interpolation.

The two-dimensional plantar pressure measurements are spatially aligned **112** by means of a rigid transformation. The spatial alignment **112** of the two-dimensional plantar pressure measurements may include generating a peak-pressure image from each of the time-varying two-dimensional plantar pressure measurements, and deriving parameters of the rigid transformation from a spatial relationship between the peak-pressure images. The desired "image" is then obtained **113** by calculating an average image from the spatially aligned two-dimensional plantar pressure measurements.

Once the images of respective subjects are obtained, they are spatially registered **130** (e.g., by using an affine transformation) and temporally registered **140** (e.g., by using continuous dynamic time warping as described above) to a target image.

The target image may be obtained **120** by first selecting **121** an intermediate target image, e.g. by random selection. For each image, a registration transformation that registers the image to the intermediate target image is determined **122**. From the images registered to the intermediate target image, an average image is calculated **123**, which will be referred to as the "biased average image". The transformations required to register the images to the intermediate target image are averaged out **124** to obtain an average transformation. This average transformation is then inverted **125** and applied **126** to the biased average image to remove the bias and obtain an unbiased target image.

Embodiments of the present invention are based on the insight of the inventors that by normalizing the pressure images to the average pressure, and by geometrically transforming the images to match a common reference image, covariates like age, height, and weight of the subjects can be omitted from the statistical modelling, which leads to more efficient processing. Accordingly, each plantar pressure image may be normalized by the total mean pressure to reduce the influence of walking speed and subject weight on the magnitude (but not the pattern) of the plantar pressure values. This normalization involves dividing each plantar pressure measurement by the sum of all pixel values in the mean pressure image.

As a result of avoiding subsampling, it is possible to locate, both in space and in time, the presence of statistically significant plantar pressure results. These results may advantageously be used to define clusters. Accordingly, a plurality of categories of subjects is derived **150** by applying a clustering algorithm to sets of data points obtained at corresponding coordinates in the spatially and temporally registered images. The registration step leads to a set of pressure data points, for each of the subjects at each of the standardized coordinates in space and time. Hence, for each of the standardized coordinates in space and time, the distribution of pressure values can be statistically analyzed. Such an analysis may lead to the identification of outliers, multiple modi, and other characteristics that allow a separation of the values into clusters.

Clustering occurring at particular standardized coordinates in space and time may coincide with clustering occurring at other standardized coordinates in space and time, leading to the identification of macroscopic phenomena (e.g., particular zones of the foot that display non-normal pressure levels in a particular phase of the footstep) that are characteristic of particular subject types (e.g., the group of subject having a particular anatomical abnormality). While a clustering algorithm may autonomously detect these clusters and generate categories accordingly, it is advantageous to record relevant known characteristics of the subjects in the expert system, such that correlations between these characteristics and the subjects' membership of particular clusters can be automatically detected by standard statistical methods (these known characteristics may include previously diagnosed medical conditions and diseases, use of certain ortheses, age group, sex, ethnicity, activity profile, etc.).

Clustering on the basis of the plantar pressure images may be refined or assisted by including other types of measurements, such as time-dependent three-dimensional scans of the foot captured in synchronization with the pressure image acquisition and force plate measurements captured in synchronization with the pressure image acquisition. Without loss of generality, the remainder of the description will focus on the use of plantar pressure images only.

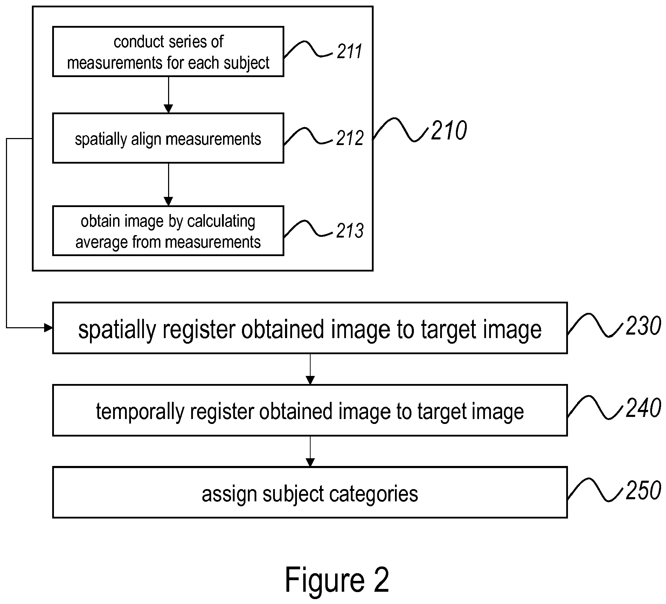

Figure 2 presents a flow chart of an embodiment of a method for using an expert system for assessing dynamic plantar pressure measurements according to the present invention. The expert system has to be trained using time-varying two-dimensional plantar pressure measurements of a number of prior subjects, preferably in accordance with the method described above with reference to Figure 1.

The method comprises obtaining **210** an image comprising time-varying two-dimensional plantar pressure measurements of the subject. Obtaining **210** the image may comprise conducting **211** a series of time-varying two-dimensional plantar pressure measurements for each subject of a group of subjects. The two-dimensional plantar pressure measurements are spatially **212** aligned. The image is obtained **213** by calculating an average image from the aligned two-dimensional plantar pressure measurements.

The obtained image is spatially registered **230** and temporally registered **240** to the target image. Data points in the spatially and temporally registered image are compared to reference values stored in the expert system to assign **250** the subject to one or more of a plurality of categories.

One or more of a treatment option, a set of shoe parameters, a set of insole parameters, a bandage type, and a medication, associated with the one or more categories, may then be outputted.

Preferably, the expert system is updated by adding the obtained image to the time-varying two-dimensional plantar pressure measurements of the number of prior subjects. The updating may be performed periodically by adding batches of obtained images to the time-varying two-dimensional plantar pressure measurements of the number of prior subjects.

The present invention also pertains to an expert system for assessing dynamic plantar pressure measurements of a subject, the expert system comprising: a learning module configured to learn by using time-varying two-dimensional plantar pressure measurements of a number of prior subjects; and an analysis module configured to analyze an image comprising time-varying two-dimensional plantar pressure measurements of a target subject; wherein the analysis module is configured to spatially and temporally register the obtained image to a target image and to compare data points in the spatially and temporally registered image to reference values stored in the expert system to assign the subject to one or more of a plurality of categories. Such an expert system may be implemented in dedicated hardware, programmable hardware (including general-purpose computers with appropriate software) or a combination of both.

The expert system and the method of using the expert system according to embodiments of the present invention may be applied by a clinician in the context of the treatment of a patient. The identification of the relevant categories to which the patient belongs may assist the clinician in making appropriate recommendations to the patient in terms of treatment and lifestyle options.

The clustering performed by the expert system allows for a distinction to be made between on the one hand natural anatomic variation or natural variation in motion, and on the other hand anatomic variation or variation in motion that must be treated and/or corrected. In particular, where a correctable deviation is detected, the output of the expert system may include a design of a personalized in-sole or orthesis, taking into account not only the outcome of the analysis of the standardized image (spatially and temporally registered and optionally normalized for total pressure), but also the parameters of the original image (foot size, shape, and position; gait pattern and asymmetries; patient weight; and the like).

A subject may be assessed at different points in time to determine the evolution of a condition. This may include performing assessment before and after a medical procedure (such as a surgical intervention), or at regular intervals throughout a rehabilitation process. The expert system thus provides a way to objectively assess the effectiveness of medical procedures aimed at restoring orthopedic functions.

The time-varying two-dimensional plantar pressure measurements obtained according to embodiments of the present invention, optionally in combination with time-dependent three-dimensional scans or force plate measurements, may be used in conjunction with a knowledge base, such as the one known from European patent application publication no. EP 1 863 385 A2, in the name of Rsscan International, to infer additional information about a part of the skeleton. The time-varying two-dimensional plantar pressure measurements obtained according to embodiments of the present invention may also be combined with additional information such as camera footage to further refine the classification performed by the expert system.

The present invention also pertains to a computer program product comprising code means configured to make a processor carry out the steps of the method of training an expert system and/or the method of using an expert system as described above. Such a computer program product may comprise code means stored on a non-volatile digital storage medium. The computer program product may be deployed as a Software-as-a-Service offering.

While the invention has been described hereinabove with reference to specific embodiments, this was done to clarify and not to limit the invention, the scope of which is determined by the accompanying claims. Features and options disclosed with reference to embodiments of the methods according to the present invention are understood to apply in the same way to embodiments of the expert system and the computer program product according to the present invention.

## Claims

1. A method for training an expert system for assessing dynamic plantar pressure measurements, the method comprising:
- obtaining (110), for each subject of said group of subjects, an image comprising time-varying two-dimensional plantar pressure measurements of at least one foot;
- spatially registering (130) said obtained images of respective subjects to a target image;
- temporally registering (140) said obtained images of respective subjects to said target image; and
- deriving (150) a plurality of categories of said subjects by applying a clustering algorithm to sets of data points obtained at corresponding coordinates in said spatially and temporally registered images.

2. The method according to claim 1, further comprising:
- obtaining, for each subject of said group of subjects, a three-dimensional scan of said at least one foot during said obtaining (110) of said image;
- spatially registering said obtained three-dimensional scans of respective subjects to a target model;
- temporally registering said obtained three-dimensional scans of respective subjects to said target model; and
- performing said deriving (150) of said plurality of categories of said subjects by applying said clustering algorithm also to sets of features in said spatially and temporally registered three-dimensional scans.

3. The method according to claim 1 or claim 2, further comprising:
- obtaining, for each subject of said group of subjects, a time-dependent force plate measurement of said at least one foot during said obtaining (110) of said image;
- temporally registering said obtained time-dependent force plate measurements of respective subjects to a target measurement; and
- performing said deriving (150) of said plurality of categories of said subjects by applying said clustering algorithm also to sets of data points obtained at corresponding coordinates in temporally registered time-dependent force plate measurements.

4. The method according to any of the preceding claims, wherein said temporally registering comprises using continuous dynamic time warping.

5. The method according to any of the preceding claims, wherein said obtaining comprises:
- conducting (111) a series of time-varying two-dimensional plantar pressure measurements for each subject of said group of subjects;
- spatially aligning (112) said two-dimensional plantar pressure measurements by means of a rigid transformation; and
- obtaining (113) said image by calculating an average image from said spatially aligned two-dimensional plantar pressure measurements.

6. The method according to claim 5, wherein said spatially aligning (112) said two-dimensional plantar pressure measurements comprises generating a peak-pressure image from each of said time-varying two-dimensional plantar pressure measurements, and deriving parameters of said rigid transformation from a spatial relationship between said peak-pressure images.

7. The method according to claim 5, wherein said spatially aligning (112) said two-dimensional plantar pressure measurements comprises generating a mean-pressure image from each of said time-varying two-dimensional plantar pressure measurements, and deriving parameters of said rigid transformation from a spatial relationship between said mean-pressure images.

8. The method according to claim 5, wherein said spatially aligning (112) said two-dimensional plantar pressure measurements comprises generating an impulse image from each of said time-varying two-dimensional plantar pressure measurements, and deriving parameters of said rigid transformation from a spatial relationship between said impulse images.

9. The method according to any of the preceding claims, further comprising obtaining (120) said target image by:
- selecting (121) an intermediate target image;
- for each image, determining (122) a registration transformation that registers said image to said intermediate target image;
- calculating (123) a biased average image from said registered images;
- calculating (124) an average transformation from said registration transformations;
- inverting (125) said average transformation; and
- applying (126) said average transformation to said biased average image to obtain said target image.

10. The method according to any of the preceding claims, wherein said group of subjects comprises humans.

11. The method according to any of claims 1-9, wherein said group of subjects comprises animals.

12. The method according to any of the preceding claims, wherein said group of subjects comprises at least 1000 subjects.

13. A method of using an expert system for assessing dynamic plantar pressure measurements of a subject, said expert system having been trained using time-varying two-dimensional plantar pressure measurements of a number of prior subjects,
the method comprising:
- obtaining (210) an image comprising time-varying two-dimensional plantar pressure measurements of at least one foot of said subject;
- spatially registering (230) said obtained image to a target image;
- temporally registering (240) said obtained images to said target image; and
- assigning (250) said subject to one or more of a plurality of categories by comparing data points in said spatially and temporally registered image to reference values stored in said expert system.

14. The method according to claim 13, further comprising:
- obtaining a three-dimensional scan of said at least one foot during said obtaining (210) of said image;
- spatially registering said obtained three-dimensional scan to a target model; and
- temporally registering said obtained three-dimensional scan to said target model;
wherein said assigning (250) further comprises comparing data points in said spatially and temporally registered three-dimensional scan to reference values stored in said expert system.

15. The method according to claim 13 or claim 14, further comprising:
- obtaining a time-dependent force plate measurement of said at least one foot during said obtaining (210) of said image; and
- temporally registering said obtained time-dependent force plate measurement to a target measurement;
wherein said assigning (250) further comprises comparing data points obtained at corresponding coordinates in temporally registered time-dependent force plate measurements to reference values stored in said expert system.

16. The method according to any of claims 13-15, further comprising: outputting one or more of a treatment option, a set of shoe parameters, a set of insole parameters, a bandage type, and a medication, associated with said one or more categories.

17. The method according to any of claims 13-16, wherein said obtaining (210) comprises:
- conducting (211) a series of time-varying two-dimensional plantar pressure measurements for each subject of a group of subjects;
- spatially (212) aligning said two-dimensional plantar pressure measurements; and
- obtaining (213) said image by calculating an average image from said aligned two-dimensional plantar pressure measurements.

18. The method according to any of claims 13 to 17, further comprising updating said expert system by adding said obtained image to said time-varying two-dimensional plantar pressure measurements of said number of prior subjects.

19. The method according to claim 18, wherein said updating is performed periodically by adding batches of obtained images to said time-varying two-dimensional plantar pressure measurements of said number of prior subjects.

20. The method according to claim 18, wherein said updating is performed by adding batches of obtained images to said time-varying two-dimensional plantar pressure measurements of said number of prior subjects, when a criterion pertaining to said obtained images is met.

21. A computer program product comprising code means configured to make a processor carry out the steps of the method of any of the preceding claims.

22. An expert system for assessing dynamic plantar pressure measurements of a subject, said expert system comprising:
- a learning module configured to learn by using time-varying two-dimensional plantar pressure measurements of a number of prior subjects; and
- an analysis module configured to analyze an image comprising time-varying two-dimensional plantar pressure measurements of a target subject;
wherein said analysis module is configured to spatially and temporally register said obtained image to a target image and to assign said subject to one or more of a plurality of categories by comparing data points in said spatially and temporally registered image to reference values stored in said expert system.
